(19) Europäisches Patentamt
European Patent Office
Office européen des brevets

(11) EP 0 676 202 B1

(12) **FASCICULE DE BREVET EUROPEEN**

(45) Date de publication et mention de la délivrance du brevet:
**07.06.2000 Bulletin 2000/23**

(51) Int Cl.[7]: **A61K 31/565**, A61K 31/58

(21) Numéro de dépôt: **95400630.0**

(22) Date de dépôt: **22.03.1995**

(54) **Application de stéroides aromatiques substitués en position 3, par une chaîne aminoalkoxy disubstituée pour obtenir un médicament destiné au contrôle de la fertilité en particulier de la fertilité masculine**

Verwendung von aromatischen Steroiden, die in der 3-Stelle bei einer disubstituierte-aminoalkoxy Kette substituiert sind, zur Herstellung eines Medikaments zur Kontrolle der Fertilität, insbesondere männlicher Fertilität

Use of aromatic steroids substituted in 3-position by a disubstituted aminoalkoxy chain for the manufacture of a medicament for the control of fertility, in particular male fertility

(84) Etats contractants désignés:
**AT BE CH DE DK ES FR GB GR IE IT LI LU NL PT SE**

(30) Priorité: **24.03.1994 FR 9403460**

(43) Date de publication de la demande:
**11.10.1995 Bulletin 1995/41**

(73) Titulaire: **HOECHST MARION ROUSSEL**
**92800 Puteaux (FR)**

(72) Inventeurs:
- **Bonfils, Armelle**
  **75019 Paris (FR)**
- **Philibert, Daniel**
  **F-94210 La Varenne Saint Hilaire (FR)**

(74) Mandataire: **Vieillefosse, Jean-Claude et al**
**Hoechst Marion Roussel**
**Département des Brevets**
**102, Route de Noisy**
**93235 Romainville Cédex (FR)**

(56) Documents cités:
**FR-A- 1 338 308** **FR-A- 2 640 977**
**FR-E- 90 803** **FR-E- 90 804**
**GB-A- 984 028** **GB-A- 984 029**

- **JOURNAL OF PHARMACY AND PHARMACOLOGY, vol. 16, no. 11, 1964 pages 717-724, D.D. EVANS ET AL. 'GENOTROPIC AGENTS : STEROID BASIC ETHERS'**
- **CHEMICAL ABSTRACTS, vol. 58, no. 12, 10 Juin 1963 Columbus, Ohio, US; abstract no. 13012, 'BIOCHEMICAL STUDIES ON DRUG METABOLISM. II' & ENDOCRINOL. JAPON., vol. 9, 1962 pages 193-200, E. TAKABATAKE ET AL.**
- **ENDOCRINOLOGY, vol. 84, no. 5, 1969 pages 1247-1249, C. LIARAKOS ET AL. 'ESTROGENIC ACTIVITIES OF SOME 3-ALKOXYESTRA-1,3,5(10)-TRIEN-17beta-OLS'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 676 202 B1

## Description

La présente invention concerne l'application de stéroïdes aromatiques substitués en position 3 par une chaîne aminoalkyloxy disubstituée pour obtenir un médicament destiné au contrôle de la fertilité et en particulier de la fertilité masculine, et les compositions pharmaceutiques le renfermant.

Dans Journal of pharmacy and pharmacol. vol 16 n°11 (1964) 717-727, D.D Evans et al. Décrivent des composés de formule (I) selon l'invention ayant une activité estrogénique et antigonadotrope pouvant être utilisés comme contraceptif.

L'invention a pour objet l'application des composés de formule (I) :

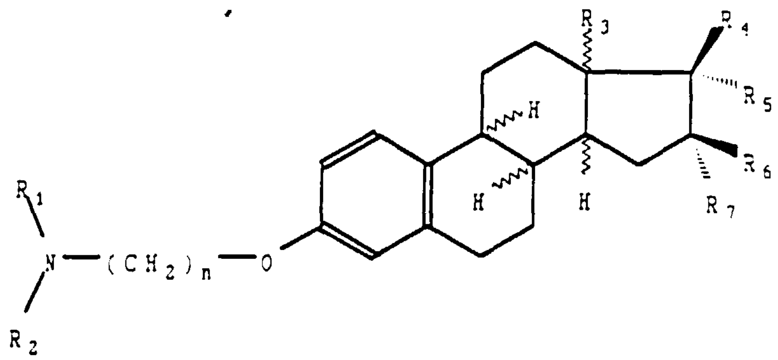

(I)

dans laquelle, $R_1$ et $R_2$, identiques ou différents, représentent un groupement alkyle ayant de 1 à 8 atomes de carbone ou un groupement benzyle, ou forment ensemble avec l'azote auquel ils sont liés un hétérocycle saturé à 5 ou 6 chaînons comportant éventuellement un autre hétéroatome choisi parmi l'oxygène et l'azote, $R_3$ en position $\alpha$ ou $\beta$ représente un groupement méthyle, n désigne un nombre entier compris entre 2 et 10, et, ou bien $R_4$ et $R_5$ forment ensemble un groupement oxo, ou $R_4$ représente un atome d'hydrogène, un groupement hydroxyle ou acyloxy ayant au plus 12 atomes de carbone et $R_5$ représente un atome d'hydrogène, un groupement hydroxyle, acyloxy ayant au plus 12 atomes de carbone, un groupement alkyle, alcényle ou alcynyle ayant chacun au plus 8 atomes de carbone, $R_6$ et $R_7$ forment ensemble un groupement oxo, ou, identiques ou différents, représentent un atome d'hydrogène, un groupement hydroxyle ou acyloxy ayant au plus 12 atomes de carbone, ou bien $R_5$ et $R_6$ forment ensemble une double liaison et $R_4$ et $R_7$ sont des atomes d'hydrogène, ainsi que leurs sels d'addition avec les acides pharmaceutiquement acceptables, pour obtenir un médicament destiné au contrôle de la fertilité et en particulier au contrôle de la fertilité masculine à l'exception de la contraception.

Lorsque $R_1$, $R_2$ et $R_5$ représentent un groupement alkyle comportant de 1 à 8 atomes de carbone il peut s'agir d'un radical méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, il s'agit de préférence de méthyle, éthyle, isopropyle.

Lorsque $R_1$ et $R_2$ forment avec l'azote auquel ils sont liés un hétérocycle saturé à 5 ou 6 chainons comportant éventuellement un autre hétéroatome choisi parmi l'oxygène et l'azote, il s'agit de préférence des groupements pipéridino, morpholino, pipérazino ou pyrrolidino.

Par groupement acyloxy ayant au plus 12 atomes de carbone, on entend de préférence des groupements choisis parmi acétyloxy, propionyloxy, butyryloxy, hexanoyloxy et benzoyloxy. On peut également citer le groupement formyloxy.

Par alcényle ayant au plus 8 atomes de carbone, on entend par exemple les valeurs suivantes :
vinyle, allyle, 1-propényle, butényle, pentényle ou hexényle.

Par alcynyle ayant au plus 8 atomes de carbone, on entend par exemple les valeurs suivantes :
éthynyle, propargyle, butynyle, pentynyle ou hexynyle.

L'invention s'étend naturellement aux sels d'addition avec les acides pharmaceutiquement acceptables des composés de formule (I), comme par exemple aux sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques. On préfére les sels d'acide chlorhydrique.

Les traits ondulés en position 8, 9 et 14 signifient que les hydrogènes sont en position ($8\beta$, $9\alpha$, $14\alpha$) ou ($8\alpha$, $9\beta$, $14\beta$).

L'invention a plus spécialement pour objet l'application précédente des composés de formule générale (I), telle que définie précédemment, dans laquelle :
$R_3$ est un groupement méthyle en position β, ainsi que de leurs sels d'addition avec les acides pharmaceutiquement acceptables.

L'invention a plus spécialement pour objet l'application précédente des composés de formule générale (I), telle que définie précédemment, répondant à la formule générale ($I_A$) :

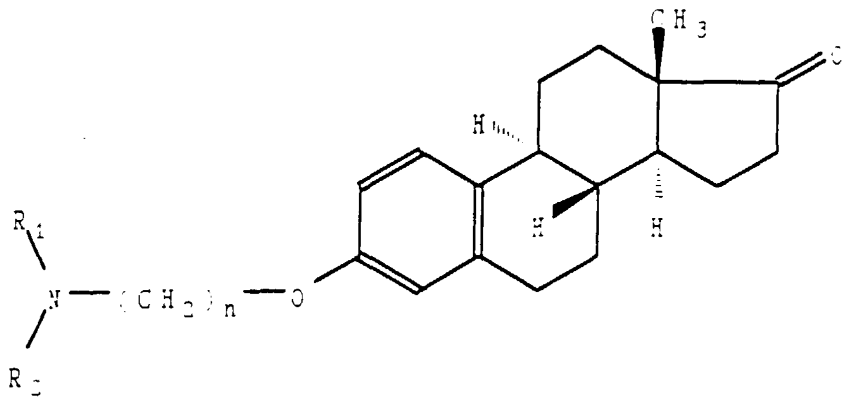
($I_A$)

dans laquelle $R_1$, $R_2$ et n sont tels que définis précédemment, ainsi que de leurs sels d'addition avec les acides pharmaceutiquement acceptables.

L'invention a plus spécialement pour objet l'application précédente des composés de formule générale (I), telle que définie précédemment, répondant à la formule générale ($I_B$) :

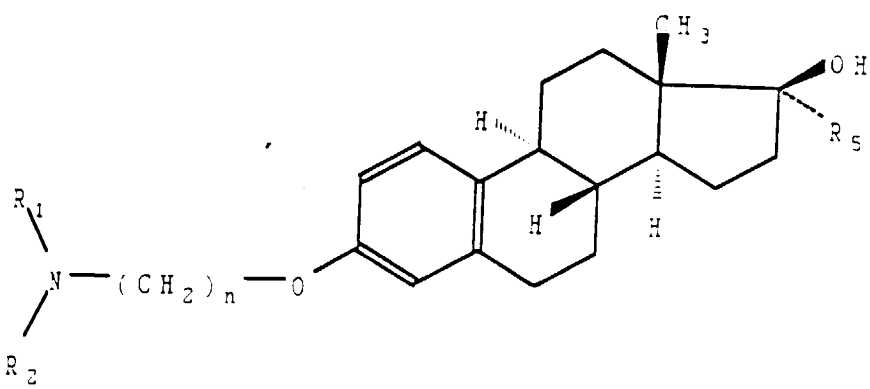
($I_B$)

dans laquelle $R_1$, $R_2$, $R_5$ et n sont tels que définis précédemment, ainsi que de leurs sels d'addition avec les acides pharmaceutiquement acceptables.

L'invention a plus spécialement pour objet l'application précédente des composés de formule générale (I), telle que définie précédemment, répondant à la formule générale ($I_C$) :

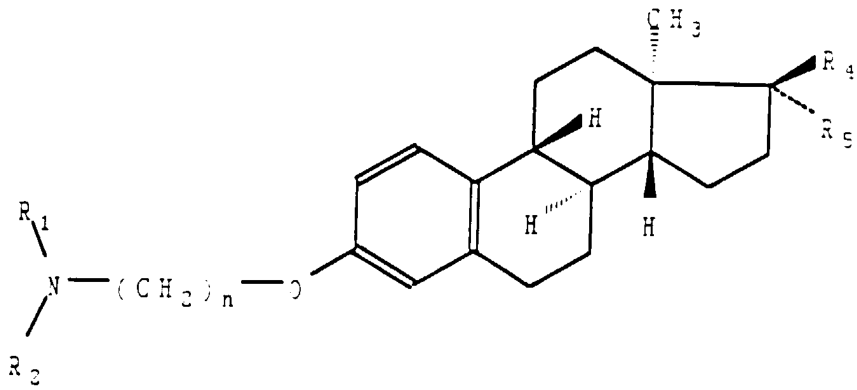

$(I_C)$

dans laquelle $R_1$ et $R_2$, identiques ou différents, représentent un groupement alkyle ayant de 1 à 8 atomes de carbone, n est tel que défini précédemment, et

a) soit $R_4$ représente un atome d'hydrogène et $R_5$ représente un groupement hydroxyle,
b) soit $R_4$ et $R_5$ forment ensemble un groupement oxo,
c) soit $R_4$ et $R_5$ représentent un atome d'hydrogène, ainsi que de leurs sels d'addition avec les acides pharmaceutiquement acceptables.

L'invention a plus spécialement pour objet l'application précédente des composés de formule générale (I), telle que définie précédemment, répondant à la formule générale ($I_D$) :

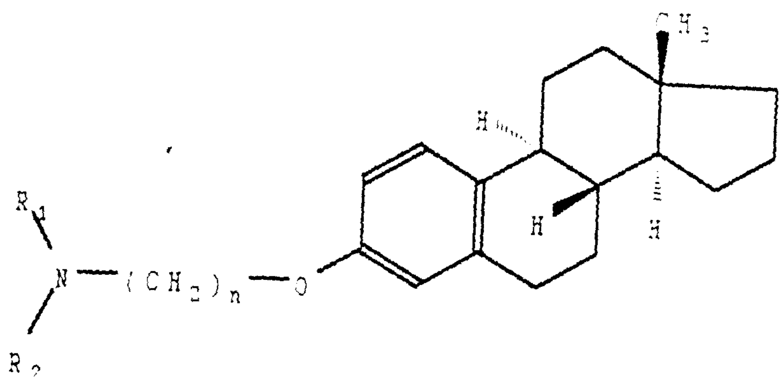

$(I_D)$

dans laquelle $R_1$, $R_2$ et n sont tels que définis précédemment, ainsi que de leurs sels d'addition avec les acides pharmaceutiquement acceptables.
L'invention a plus spécialement pour objet l'application précédente des composés de formule générale (I), telle que définie précédemment, répondant à la formule générale ($I_E$) :

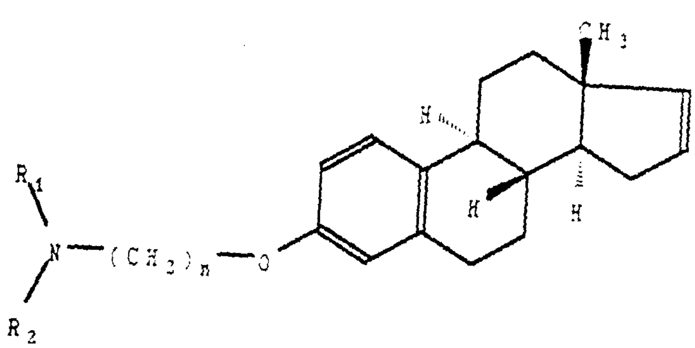

$(I_E)$

dans laquelle $R_1$, $R_2$ et n sont tels que définis précédemment, ainsi que de leurs sels d'addition avec les acides pharmaceutiquement acceptables.

L'invention a plus spécialement pour objet l'application précédente des composés de formule générale (I) telle que définie précédemment, répondant à la formule générale ($I_F$) :

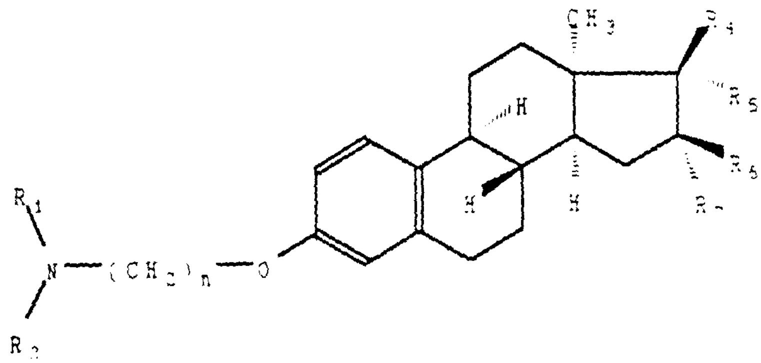

($I_F$)

dans laquelle $R_1$, $R_2$ et n sont tels que définis précédemment, et, ou bien, $R_4$ et $R_5$ forment ensemble un groupement oxo, ou, identiques ou différents, représentent un atome d'hydrogène, un groupement hydroxyle ou acyloxy ayant au plus 12 atomes de carbone, $R_6$ et $R_7$ forment ensemble un groupement oxo, ou, identiques ou différents représentent un atome d'hydrogène, un groupement hydroxyle ou acyloxy ayant au plus 12 atomes de carbone, ou bien $R_5$ et $R_6$ forment ensemble une double liaison et $R_4$ et $R_7$ sont des atomes d'hydrogène, ainsi que de leurs sels d'addition avec les acides pharmaceutiquement acceptables.

L'invention a plus spécialement pour objet l'application précédente des composés de formule générale (I), telle que définie précédemment, dans laquelle $R_1$ et $R_2$ identiques représentent un méthyle ou un éthyle, ainsi que de leurs sels d'addition avec les acides pharmaceutiquement acceptables.

L'invention a plus spécialement pour objet l'application précédente des composés de formule générale (I), telle que définie précédemment, dans laquelle n est égal à 2, ainsi que de leurs sels d'addition avec les acides pharmaceutiquement acceptables.

L'invention a tout spécialement pour objet l'application précédente des composés de formule générale (I) suivants :

- 3-[2-(diméthylamino) éthoxy]-estra-1,3,5(10)-trièn-17-one,
- 3-[2-(diéthylamino) éthoxy]-estra-1,3,5(10)-trièn-17-one,
- chlorhydrate de 3-[2-(diméthylamino) éthoxy]-estra 1,3,5(10)-trièn-17-one,
- chlorhydrate de 3-[2-(diéthylamino) éthoxy]-estra-1,3,5(10)-trièn-17-one,
- 3-[2-(1-pipéridinyl) éthoxy]-estra-1,3,5(10)-trièn-17-one,
- 3-[2-(4-morpholinyl) éthoxy]-estra-1,3,5(10)-trièn-17-one,
- 3-[3-(diméthylamino) propoxy]-estra-1,3,5(10)-trièn-17-one,
- 3-[3-(diéthylamino) propoxy]-estra-1,3,5(10)-trièn-17-one,
- 3-[4-(diméthylamino) butoxy]-estra-1,3,5(10)-trièn-17-one,
- 3-[5-(diméthylamino) pentyloxy]-estra-1,3,5(10)-trièn-17-one,
- 3-[5-(diéthylamino) pentyloxy]-estra-1,3,5(10)-trièn-17-one,
- 3-[10-(diméthylamino) decyloxy]-estra-1,3,5(10)-trièn-17-one,
- 3-[2-[méthyl(phénylméthyl) amino] ethoxy]-estra-1,3,5(10)-triène-17-one,
- 3-[3-[bis(phénylméthyl) amino] propoxy]-estra-1,3,5(10)-trièn-17-one,
- (17β)-3-[2-[méthyl(phénylméthyl) amino] éthoxy]-estra-1,3,5(10)-trièn-17-ol,
- (17β)-3-[3-(diméthylamino) propoxy]-estra-1,3,5(10)trièn-17-ol,
- (17β)-3-[3-(diéthylamino)propoxy]-estra-1,3,5(10)trièn-17-ol,
- (17α)-3-[3-(diéthylamino) propoxy]-estra-1,3,5(10)-trièn-17-ol,
- (17α)-3-[2-(diméthylamino) éthoxy]-estra-1,3,5(10)-trièn-17-ol,
- (17α)-3-[2-(diéthylamino) éthoxy]-estra-1,3,5(10)-trièn-17-ol,
- 3-[2-(diméthylamino) éthoxy]-estra-1,3,5(10)-trièn-16-one,
- 3-[2-(diéthylamino) éthoxy]-estra-1,3,5(10)-trièn-16-one,
- chlorhydrate de 3-[2-(diéthylamino) éthoxy]-estra-1,3,5(10)-trièn-16-one,
- 3-[2-(diméthylamino) éthoxy]-estra-1,3,5(10)-triène,
- 3-[2-(diéthylamino) éthoxy]-estra-1,3,5(10)-triène,

- 3-[2-(1-pipéridinyl) éthoxy]-estra-1,3,5(10)-triène,
- 3-[3-(1-pipéridinyl) propoxy]-estra-1,3,5(10)-triène,
- chlorhydrate de 3-[2-(diéthylamino) éthoxy]-estra-1,3,5(10)-triène
- 3-[2-(4-morpholinyl) éthoxy]-estra-1,3,5(10)-triène,
- 3-[2-(diméthylamino) éthoxy)]-estra-1,3,5(10),16-tétraène,
- 3-[2-(diéthylamino)éthoxy)]-estra-1,3,5(10),16-tétraène,
- (17α)-3-[3-(diméthylamino) propoxy]-17-methyl-estra-1,3,5(10)-trièn-17-ol,
- (17α)-3-[2-(diméthylamino) éthoxy]-17-methyl-estra-1,3,5(10)-trièn-17-ol,
- (17α)-3-[3-(diméthylamino) propoxy]-19-nor-pregna-1,3,5(10)-trièn-17-ol,
- (17α)-3-[2-(diméthylamino) éthoxy]-19-nor-pregna-1,3,5(10)-trièn-17-ol,
- (17α)-3-[2-[méthyl(phénylméthyl)amino]éthoxy]-19-nor-pregna-1,3,5(10)-trièn-17-ol,
- (17α)-3-[3-(diméthylamino) propoxy]-17-propyl-estra-1,3,5(10)-trièn-17-ol,
- chlorhydrate de (17α)-3-[3-(diméthylamino)propoxy]-17-propyl-estra-1,3,5(10)-trièn-17-ol,
- (17α)-3-[3-(diméthylamino)propoxy]-17-(1-propényl)-estra-1,3,5(10)-trièn-17-ol,
- chlorhydrate de (17α)-3-[3-(diméthylamino)propoxy]-17-(1-propényl)-estra-1,3,5(10)-trièn-17-ol,
- (17α)-3-[2-(diméthylamino)éthoxy)]-19-nor-pregna-1,3,5(10),20-tétraèn-17-ol,
- (17α)-3-[3-(diméthylamino)propoxy]-19-nor-pregna-1,3,5(10),20-tétraèn-17-ol,
- (17α)-3-[2-(diméthylamino)éthoxy]-19-nor-pregna-1,3,5(10)-trièn-20-yn-17-ol,
- (17α)-3-[3-(diméthylamino)propoxy]-19-nor-pregna-1,3,5(10)-trièn-20-yn-17-ol,
- chlorhydrate de (17α)-3-[3-(diméthylamino)propoxy]-19-nor-pregna-1,3,5(10)-trièn-20-yn-17-ol,
- (17α)-3-[2-(diéthylamino) éthoxy]-19-nor-pregna-1,3,5(10)-trièn-20-yn-17-ol,
- (17α)-3-[3-(diéthylamino) propoxy]-19-nor-pregna-1,3,5(10)-trièn-20-yn-17-ol,
- (17α)-3-[2-[méthyl(phénylméthyl)amino]éthoxy]-19-nor-pregna-1,3,5(10)-trièn-20-yn-17-ol,
- chlorhydrate de (17α)-3-[2-[méthyl(phénylméthyl) amino] éthoxy]-19-nor-pregna-1,3,5(10)-trièn-20-yn-17-ol,
- (17α)-3-[3-[méthyl(phénylméthyl) amino] propoxy]-19-nor-pregna-1,3,5(10)-trièn-20-yn-17-ol,
- (8α,9β,13α,14β)-3-[2-(diméthylamino)éthoxy]-estra-1,3,5(10)-trièn-17-one,
- (8α,9β,13α,14β)-3-[2-(diéthylamino)éthoxy]-estra-1,3,5(10)-trièn-17-one,
- (8α,9β,13α,14β)-3-[2-(diméthylamino)éthoxy]-estra-1,3,5(10)-triène,
- (8α,9β,13α,14β)-3-[2-(diéthylamino)éthoxy]-estra-1,3,5(10)-triène,
- chlorhydrate de (8α,9β,13α,14β)-3-[2(diéthylamino)éthoxy]-estra-1,3,5(10)-triène,
- (8α,9β,13α,14β,17α)-3-[2-(diéthylamino)éthoxy]-estra-1,3,5(10)-trièn-17-ol,
- (13α)-3-[2-(diméthylamino)éthoxy]-estra-1,3,5(10)-trièn-17-one,
- (13α)-3-[2-(diéthylamino) éthoxy]-estra-1,3,5(10)-trièn-17-one,
- (13α)-3-[2-(diméthylamino)éthoxy]-estra-1,3,5(10)-triène,
- (13α)-3-[2-(diéthylamino)éthoxy]-estra-1,3,5(10)-triène.

La synthèse des produits de formule (I) est décrite dans le brevet Français 1.338.308 et les brevets d'addition 90803 et 90804, le brevet Américain 3,212,971 et dans les brevets Britanniques 984,028 et 984,029.
Les produits de formule (I) sont connus pour leurs propriétés antilipémiques ou hypocholestérolémiques.
La demanderesse a mis en évidence leurs nouvelles propriétés dans la régulation du flux calcique dans le spermatozoïde.
Les produits de l'invention présentent

1) une forte affinité pour les récepteurs Sigma (voir tests pharmacologiques)
2) une activité vis-à-vis de l'influx du calcium dans le spermatozoïde.

Les résultats des tests montrent que parmi les produits se fixant sur les récepteurs Sigma, certains agissent en stimulant l'influx de calcium dans le spermatozoïde, d'autres en inhibant l'influx du calcium stimulé ou non par la progestérone, molécule décrite comme se liant au récepteur Sigma.
Les produits de formule (I), ayant une activité agoniste, stimulent l'influx du calcium dans le spermatozoïde. Ils peuvent donc être utilisés dans le traitement de certaines formes de stérilité caractérisées par un pouvoir fécondant insuffisant des spermatozoïdes.
Les produits de formule (I) ayant une activité antagoniste, inhibent l'influx du calcium dans le spermatozoïde. Ils sont donc potentiellement utilisables dans le contrôle de la réaction acrosomiale et interviennent par conséquent sur le pouvoir fécondant du spermatozoïde. Ils peuvent donc être utilisés comme contraceptif et en particulier comme contraceptif masculin.
Ils peuvent aussi être utilisés dans le domaine vétérinaire comme contraceptif mâle chez les animaux de compagnie (chiens, chats ...) ou pour limiter la prolifération de toute espèce nuisible, en particulier les rongeurs ou les

pigeons.

La posologie utile varie en fonction de l'affection à traiter et de la voie d'administration. Elle peut varier par exemple de 10 à 1000 mg par jour chez l'homme adulte par voie orale.

L'invention a également pour objet l'application des composés de formule (I) tels que définis précédemment à titre de contraceptif masculin étant entendu que l'utilisation de la contraception à titre privé est exclue.

**TESTS PHARMACOLOGIQUES**

**METHODE**

Préparation des spermatozoïdes humains

Le sperme humain provient de donneurs sains. Les spermatozoïdes mobiles sont séparés par centrifugation sur gradient de Percoll (47,5-95 %) puis remis en suspension dans un milieu BWW (Biggers whitten and whittinghan) hypertonique contenant : NaCl 166 mM, KCl 5 mM, $CaCl_2$ 1,3 mM, $KH_2PO_4$ 1,2 mM, $MgSO_4$ 1,2 mM, glucose 5,5 mM, lactate de sodium 21 mM, pyruvate de sodium 0,25 mM, $NaHCO_3$ 25 mM, Hepes 20 mM et 0,8 % de HSA (Human serum Albumin) (410 mosm/litre), pH 7,4 à température ambiante.

Mesure du calcium intracellulaire

Les spermatozoïdes mobiles sont incubés au minimum pendant 2 heures dans le milieu capacitant BWW/HSA. Ils sont ensuite incubés (à une concentration de 5-10 x $10^6$/ml) avec du Fura2-AM (concentration finale 2 µM) à 37°C pendant 45 minutes. Après lavage par centrifugation pendant 10 mn dans du BWW sans HSA, les spermatozoïdes sont remis en suspension à une concentration de 4 x $10^6$/ml. Le signal de fluorescence est mesuré à 37°C à l'aide d'un spectrofluorimètre à des longueurs d'onde d'excitation de 340 et 380 nm (PTIM 2001-Kontron) ou à 340, 360 et 380 nm (Hitachi F 2000 - B. Braun Science Tec.). L'émission de fluorescence est enregistrée à 505 nm. La progestérone ou les produits à tester, dissous dans l'éthanol absolu, sont ajoutés au milieu d'incubation à une concentration finale de 0,1 % d'éthanol. Lorsqu'un effet antagoniste de la progestérone est recherché, le produit est ajouté au milieu 2 mn avant la progestérone. A la fin de chaque dosage, 5 µM de ionomycine sont ajoutés à l'échantillon afin de mesurer le signal de fluorescence maximum ; puis les spermatozoïdes sont perméabilisés avec 0,05 % de Triton X-100 et 10 mM d'EGTA sont ajoutés (pH 9,5) afin de mesurer le signal de fluorescence minimum. Ces valeurs permettent de calculer la concentration intracellulaire en calcium ($[Ca^{2+}]i$) selon la méthode décrite par Grunkiewicz at al (Grunkiewicz G., Poenie M. and Tsien R.Y. (1985) J. Biol. Chem. 260, 3440-3450). Les résultats des concentrations en calcium intracellulaire sont exprimés par rapport au nivau basal pris arbitrairement égal à 1.

Récepteur sigma : mesure de l'affinité relative de liaison

L'affinité relative de liaison est évaluée sur des préparations de membranes de cerveau et de testicules de rat. Préparation des membranes :

Des rats mâles Sprague-Dawley provenant d'Iffa Credo et pesant environ 200 g sont utilisés. Les animaux sont sacrifiés par décapitation, le cerveau et les testicules sont prélevés et homogénéisés dans 10 à 25 volumes de tampon Tris-HCl 50 mM (pH 7,7) à 4°C, à l'aide d'un Ultrathurax. Les homogénats sont centrifugés à 30 000 g pendant 15 mn à 4°C, les culots sont ensuite lavés 3 fois par remise en suspension (dans le même tampon) et centrifugation dans les mêmes conditions. Les membranes ainsi obtenues sont conservées à -80°C.

**Incubation :**

Le marqueur des récepteurs sigma utilisé est le $^3H$ PPP (propyl-3-(3-hydroxyphényl) pipéridine) de NEN (New England Nuclear) possédant une activité spécifique de 3404 GBq/mmol.

Les membranes sont remises en suspension dans du tampon Tris-HCl 50 mM, pH 8,0 de façon à avoir une concentration en protéines d'environ 0,6 mg/ml pour les testicules et 1 mg/ml pour le cerveau. Des aliquotes d'homogénat sont incubées pendant 90 mn à 25°C (dans un volume total de 0,5 ml) avec 3nM de $^3H$ PPP en présence de concentrations croissantes de produit de référence (halopéridol) ou des produits à tester. A la fin de l'incubation, le $^3H$ PPP lié aux membranes est séparé du $^3H$ PPP libre par filtration rapide sur des filtres Whatman GF/C préalablement prétraités avec 0,05 % de poly-éthylèneimine. Le précipité est ensuite lavé 2 fois avec 5 ml de tampon Tris-HCl. Le comptage de la radioactivité est effectué après addition de 20 ml de liquide scintillant Aqualyte (Baker).

**Calcul de l'affinité relative de liaison (ARL) :**

Les deux courbes suivantes sont tracées : pourcentage du marqueur tritié lié 100 x B/BO en fonction du logarithme de la concentration du produit de référence froid ou en fonction du logarithme de la concentration du produit froid testé. La droite d'équation suivante est déterminée :

$$I50 = 100(BO/BO+Bmin/BO)/2$$

soit

$$I50=100\ (1+Bmin/BO)/2 = 50(1+Bmin/BO)$$

BO = concentration du marqueur tritié lié en l'absence de tout produit froid.
B = concentration du marqueur tritié lié en présence d'une concentration X de produit froid.
Bmin = concentration du marqueur tritié lié en présence d'un grand excès du produit de référence froid (5 000 nM).

Les intersections de la droite I50 et des courbes, permettent d'évaluer les concentrations du produit de référence froid (CH) et du produit froid testé (CX) qui inhibent de 50 % la liaison spécifique du marqueur tritié sur le récepteur. L'affinité relative de liaison (ARL) du produit testé est déterminée par l'équation :

$$ARL = 100\ (CH)/(CX).$$

L'ARL de l'halopéridol est prise arbitrairement égale à 100.

Affinité relative de liaison pour le récepteur Sigma

| | Cerveau (rat) | Testicule (rat) |
|---|---|---|
| Réf : Halopéridol | 100,0 | 100,0 |
| Progestérone | 0,7 | 0,3 |
| Estrone | < 0,06 | |
| (8α,9β,13α,14β)-3-[2-(diméthylamino)éthoxy]-estra-1,3,5(10)-triène (pdt X) | 23,0 | 0,5 |
| 3-[2-(diéthylamino)éthoxy]-estra-1,3,5(10)-trièn-17-one (pdt W) | 85,0 | 12,5 |
| 3-[2-(1-pipéridinyl)éthoxy]-estra-1,3,5(10)-trièn-17-one | 28,0 | |
| (13α)-3-[2-(diéthylamino)éthoxy]-estra-1,3,5(10)-trièn-17-one | 39, 0 | |
| 3-[2-(1-pipéridinyl)éthoxy]-estra-1,3,5(10)-triène | 54,0 | |
| chlorhydrate de (8α,9β,13α,14β)-3-[2(diéthylamino)éthoxy]-estra-1,3,5(10)-triène | 28,0 | |
| (17α)-3-[2-(diéthylamino)éthoxy]-19-nor-pregna-1,3,5(10)-trièn-20-yn-17-ol | 24, 0 | |

Augmentation de la concentration en calcium intracellulaire ([$Ca^{2+}$]i) induit par la progestérone et le produit W

| | Progestérone ($10^{-5}$M) | Produit W ($10^{-5}$M) |
|---|---|---|
| Exp. 1 | 4,25 | 3,56 |
| Exp. 2 | 6,60 | 2,55 |

Ces résultats sont exprimés par rapport au niveau basal pris égal à 1.

Les niveaux de base initiaux sont :

Exp. 1 = 200 nm et Exp. 2 = 250 nm.

Effet dose du produit X sur ([$Ca^{2+}$]i)

| PRODUIT X | | | | | |
|---|---|---|---|---|---|
| | $10^{-7}$ M | $10^{-6}$ M | $5x10^{-6}$ M | $10^{-5}$ M | $2x10^{-5}$ M |
| Exp. 1 | 1,50 | 1,63 | 2,55 | 7,20 | 8,50 |
| Exp. 2 | 1,38 | 1,78 | 5,56 | 12,00 | 8,00 |

Les résultats sont exprimés par rapport au niveau basal pris égal à 1.

Comparaison des effets de la progestérone à $10^{-5}$ M et du produit X à $10^{-5}$ M sur [$Ca^{2+}$]i Mean ± SEM n = 3

| Progestérone | Produit X |
|---|---|
| 4,90 ± 0,92 | 4,80 ± 0,92 |

Les résultats sont exprimés par rapport au niveau basal pris égal à 1.

**CONCLUSION :**

Effet sur le calcium intracellulaire des spermatozoïdes humains

La progestérone à la concentration de $10^{-5}$ M induit une augmentation transitoire du [$Ca^{2+}$]i suivie d'une deuxième phase ou le [$Ca^{2+}$]i est légèrement supérieur au niveau basal.

Le produit X provoque une augmentation dose-dépendante du [$Ca^{2+}$]i qui atteint à $10^{-5}$ M une intensité égale à celle provoquée par la progestérone. Contrairement à ce que l'on observe avec la progestérone, cet effet est prolongé dans le temps.

Affinité relative de liaison (ARL) pour le récepteur sigma

Ce produit ainsi que la progestérone, sont capables de déplacer le $^{3}$H PPP. Les ARLs calculées sur des membranes de cerveau de rat ont également été évaluées sur testicules et sont données dans le tableau.

Les différences observées entre les ARL au niveau du cerveau et des testicules pourraient s'expliquer par une répartition différente des divers types de sites du récepteur sigma entre ces deux organes.

De tels produits peuvent donc stimuler la réaction acrosomiale dans le cas des agonistes comme le produit X et donc être utilisés dans certaines formes de stérilité caractérisées par un pouvoir fécondant insuffisant des spermatozoïdes.

**Revendications**

1. L'application des composés de formule (I) :

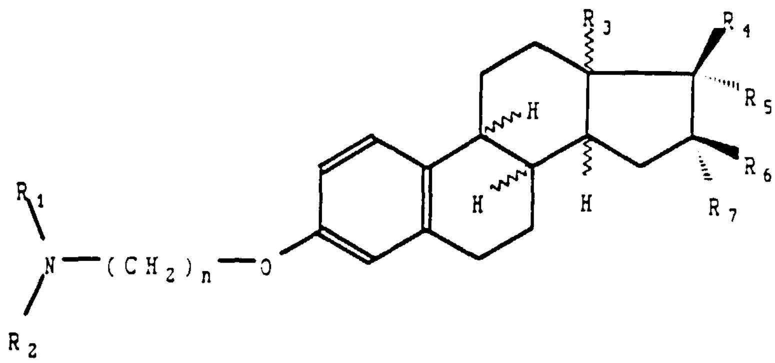

(I)

dans laquelle, $R_1$ et $R_2$, identiques ou différents, représentent un groupement alkyle ayant de 1 à 8 atomes de carbone ou un groupement benzyle, ou forment ensemble avec l'azote auquel ils sont liés un hétérocycle saturé à 5 ou 6 chaînons comportant éventuellement un autre hétéroatome choisi parmi l'oxygène et l'azote, $R_3$ en position $\alpha$ ou $\beta$ représente un groupement méthyle, n désigne un nombre entier compris entre 2 et 10, et, ou bien $R_4$ et $R_5$ forment ensemble un groupement oxo, ou $R_4$ représente un atome d'hydrogène, un groupement hydroxyle ou acyloxy ayant au plus 12 atomes de carbone et $R_5$ représente un atome d'hydrogène, un groupement hydroxyle, acyloxy ayant au plus 12 atomes de carbone, alkyle, alcényle ou alcynyle ayant chacun au plus 8 atomes de carbone, $R_6$ et $R_7$ forment ensemble un groupement oxo, ou, identiques ou différents, représentent un atome d'hydrogène, un groupement hydroxyle ou acyloxy ayant au plus 12 atomes de carbone, ou bien $R_5$ et $R_6$ forment ensemble une double liaison et $R_4$ et $R_7$ sont des atomes d'hydrogène, ainsi que leurs sels d'addition avec les acides pharmaceutiquement acceptables, pour obtenir un médicament destiné au contrôle de la fertilité et en particulier au contrôle de la fertilité masculine à l'exception de la contraception.

**2.** L'application selon la revendication 1 des composés de formule générale (I), telle que définie à la revendication 1, dans laquelle :
$R_3$ est un groupement méthyle en position $\beta$, ainsi que de leurs sels d'addition avec les acides pharmaceutiquement acceptables.

**3.** L'application selon la revendication 1 des composés de formule générale (I), telle que définie à la revendication 1 ou 2, répondant à la formule générale ($I_A$) :

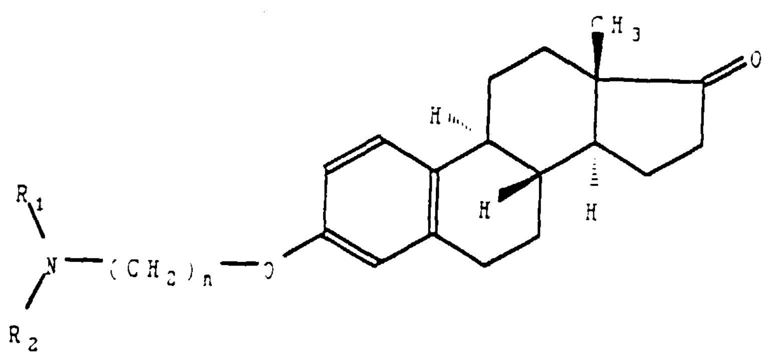

($I_A$)

dans laquelle $R_1$, $R_2$ et n sont tels que définis à la revendication 1, ainsi que de leurs sels d'addition avec les acides pharmaceutiquement acceptables.

**4.** L'application selon la revendication 1 des composés de formule générale (I), telle que définie à la revendication 1 ou 2, répondant à la formule générale ($I_B$) :

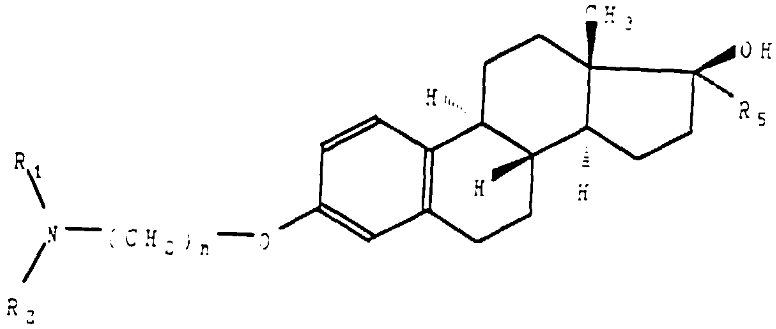

$(I_B)$

dans laquelle $R_1$, $R_2$, $R_5$ et n sont tels que définis à la revendication 1, ainsi que de leurs sels d'addition avec les acides pharmaceutiquement acceptables.

**5.** L'application selon la revendication 1 des composés de formule générale (I) telle que définie à la revendication 1, répondant à la formule générale $(I_C)$ :

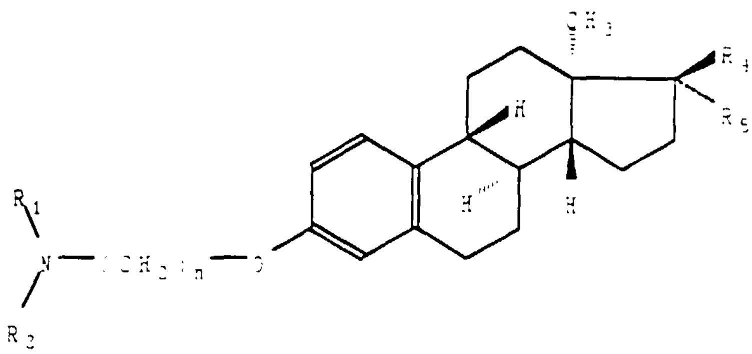

$(I_C)$

dans laquelle $R_1$ et $R_2$, identiques ou différents, représentent un groupement alkyle ayant de 1 à 8 atomes de carbone, n est tel que défini précédemment, et

a) <u>soit</u> $R_4$ représente un atome d'hydrogène et $R_5$ représente un groupement hydroxyle,
b) <u>soit</u> $R_4$ et $R_5$ forment ensemble un groupement oxo,
c) <u>soit</u> $R_4$ et $R_5$ représentent un atome d'hydrogène,

ainsi que de leurs sels d'addition avec les acides pharmaceutiquement acceptables.

**6.** L'application selon la revendication 1 des composés de formule générale (I), telle que définie à la revendication 1 ou 2, répondant à la formule générale $(I_D)$ :

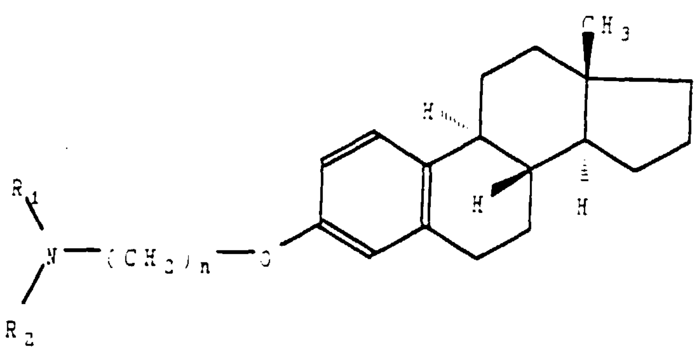

$(I_D)$

dans laquelle $R_1$, $R_2$ et n sont tels que définis à la revendication 1 ainsi que de leurs sels d'addition avec les acides pharmaceutiquement acceptables.

**7.** L'application selon la revendication 1 des composés de formule générale (I) telle que définie à la revendication 1 ou 2, répondant à la formule générale ($I_E$) :

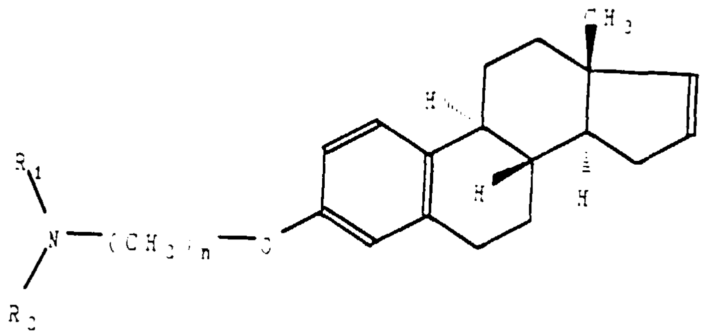

($I_E$)

dans laquelle $R_1$, $R_2$ et n sont tels que définis à la revendication 1 ainsi que de leurs sels d'addition avec les acides pharmaceutiquement acceptables.

**8.** L'application selon la revendication 1 des composés de formule générale (I) telle que définie à la revendication 1, répondant à la formule générale ($I_F$) :

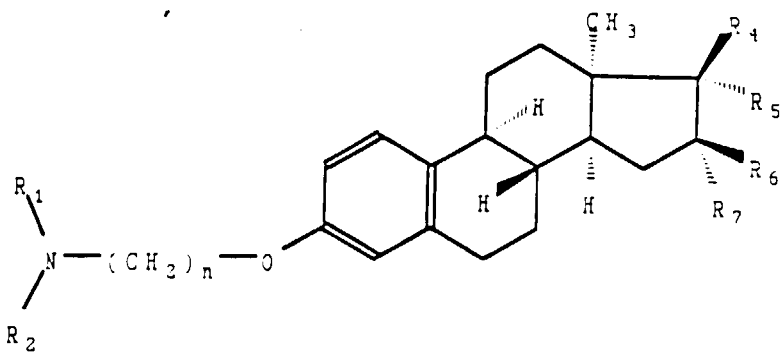

($I_F$)

dans laquelle $R_1$, $R_2$ et n sont tels que définis à la revendication 1, et, <u>ou bien,</u> $R_4$ et $R_5$ forment ensemble un groupement oxo, ou, identiques ou différents, représentent un atome d'hydrogène, un groupement hydroxyle ou acyloxy ayant au plus 12 atomes de carbone, $R_6$ et $R_7$ forment ensemble un groupement oxo, ou, identiques ou différents représentent un atome d'hydrogène, un groupement hydroxyle ou acyloxy ayant au plus 12 atomes de carbone, <u>ou bien</u> $R_5$ et $R_6$ forment ensemble une double liaison et $R_4$ et $R_7$ sont des atomes d'hydrogène, ainsi que de leurs sels d'addition avec les acides pharmaceutiquement acceptables.

**9.** L'application selon la revendication 1 des composés de formule générale (I), telle que définie à l'une quelconque des revendications 1 à 8, dans laquelle $R_1$ et $R_2$, identiques, représentent un méthyle ou un éthyle, ainsi que de leurs sels d'addition avec les acides pharmaceutiquement acceptables.

**10.** L'application selon la revendication 1 des composés de formule générale (I), telle que définie à l'une quelconque des revendications 1 à 9, dans laquelle n est égal à 2, ainsi que de leurs sels d'addition avec les acides pharmaceutiquement acceptables.

**11.** L'application selon la revendication 1 des composés de formule générale (I) telle que définie à la revendication 1, suivants :

- 3-[2-(diméthylamino) éthoxy]-estra-1,3,5(10)-trièn-17-one,

- 3-[2-(diéthylamino) éthoxy]-estra-1,3,5(10)-trièn-17-one,
- chlorhydrate de 3-[2-(diméthylamino) éthoxy]-estra-1,3,5(10)-trièn-17-one,
- chlorhydrate de 3-[2-(diéthylamino) éthoxy]-estra-1,3,5(10)-trièn-17-one,
- 3-[2-(1-pipéridinyl) éthoxy]-estra-1,3,5(10)-trièn-17-one,
- 3-[2-(4-morpholinyl) éthoxy]-estra-1,3,5(10)-trièn-17-one,
- 3-[3-(diméthylamino) propoxy]-estra-1,3,5(10)-trièn-17-one,
- 3-[3-(diéthylamino) propoxy]-estra-1,3,5(10)-trièn-17-one,
- 3-[4-(diméthylamino) butoxy]-estra-1,3,5(10)-trièn-17-one,
- 3-[5-(diméthylamino) pentyloxy]-estra-1,3,5(10)-trièn-17-one,
- 3-[5-(diéthylamino) pentyloxy]-estra-1,3,5(10)-trièn-17-one,
- 3-[10-(diméthylamino) decyloxy]-estra-1,3,5(10)-trièn-17-one,
- 3-[2-[méthyl(phénylméthyl)amino]ethoxy]-estra-1,3,5(10)-triène-17-one,
- 3-[3-[bis(phénylméthyl)amino]propoxy]-estra-1,3,5(10)-trièn-17-one,
- (17β)-3-[2-[méthyl(phénylméthyl)amino]éthoxy]-estra-1,3,5(10)-trièn-17-ol,
- (17β)-3-[3-(diméthylamino)propoxy]-estra-1,3,5(10)trièn-17-ol,
- (17β)-3-[3-(diéthylamino)propoxy]-estra-1,3,5(10)trièn-17-ol,
- (17α)-3-[3-(diéthylamino)propoxy]-estra-1,3,5(10)-trièn-17-ol,
- (17α)-3-[2-(diméthylamino)éthoxy]-estra-1,3,5(10)-trièn-17-ol,
- (17α)-3-[2-(diéthylamino)éthoxy]-estra-1,3,5(10)-trièn-17-ol,
- 3-[2-(diméthylamino)éthoxy]-estra-1,3,5(10)-trièn-16-one,
- 3-[2-(diéthylamino)éthoxy]-estra-1,3,5(10)-trièn-16-one,
- chlorhydrate de 3-[2-(diéthylamino) éthoxy]-estra-1,3,5(10)-trièn-16-one,
- 3-[2-(diméthylamino) éthoxy]-estra-1,3,5(10)-triène,
- 3-[2-(diéthylamino) éthoxy]-estra-1,3,5(10)-triène,
- 3-[2-(1-pipéridinyl) éthoxy]-estra-1,3,5(10)-triène,
- 3-[3-(1-pipéridinyl) propoxy]-estra-1,3,5(10)-triène,
- chlorhydrate de 3-[2-(diéthylamino) éthoxy]-estra-1,3,5(10)-triène
- 3-[2-(4-morpholinyl) éthoxy]-estra-1,3,5(10)-triène,
- 3-[2-(diméthylamino) éthoxy)]-estra-1,3,5(10),16-tétraène,
- 3-[2-(diéthylamino) éthoxy)]-estra-1,3,5(10),16-tétraène,
- (17α)-3-[3-(diméthylamino) propoxy] -17-methyl-estra-1,3,5(10)-trièn-17-ol,
- (17α)-3-[2-(diméthylamino) éthoxy] -17-methyl-estra-1,3,5(10)-trièn-17-ol,
- (17α)-3-[3-(diméthylamino) propoxy]-19-nor-pregna-1,3,5(10)-trièn-17-ol,
- (17α) -3-[2-(diméthylamino) éthoxy]-19-nor-pregna-1,3,5(10)-trièn-17-ol,
- (17α)-3-[2-[méthyl(phénylméthyl) amino] éthoxy]-19-nor-pregna-1,3,5(10)-trièn-17-ol,
- (17α)-3-[3-(diméthylamino) propoxy]-17-propyl-estra-1,3,5(10)-trièn-17-ol,
- chlorhydrate de (17α)-3-[3-(diméthylamino) propoxy]-17-propyl-estra-1,3,5(10)-trièn-17-ol,
- (17α)-3-[3-(diméthylamino) propoxy]-17-(1-propényl)-estra-1,3,5(10)-trièn-17-ol,
- chlorhydrate de (17α)-3-[3-(diméthylamino) propoxy]-17-(1-propényl)-estra-1,3,5(10)-trièn-17-ol,
- (17α)-3-[2-(diméthylamino) éthoxy)]-19-nor-pregna-1,3,5(10),20-tétraèn-17-ol,
- (17α)-3-[3-(diméthylamino) propoxy]-19-nor-pregna-1,3,5(10),20-tétraèn-17-ol,
- (17α)-3-[2-(diméthylamino) éthoxy]-19-nor-pregna-1,3,5(10)-trièn-20-yn-17-ol,
- (17α)-3-[3-(diméthylamino) propoxy]-19-nor-pregna-1,3,5(10)-trièn-20-yn-17-ol,
- chlorhydrate de (17α)-3-[3-(diméthylamino) propoxy]-19-nor-pregna-1,3,5(10)-trièn-20-yn-17-ol,
- (17α)-3-[2-(diéthylamino) éthoxy]-19-nor-pregna-1,3,5(10)-trièn-20-yn-17-ol,
- (17α)-3-[3-(diéthylamino) propoxy]-19-nor-pregna-1,3,5(10)-trièn-20-yn-17-ol,
- (17α)-3-[2-[méthyl(phénylméthyl) amino] éthoxy]-19-nor-pregna-1,3,5(10)-trièn-20-yn-17-ol,
- chlorhydrate de (17α)-3-[2-[méthyl(phénylméthyl) amino] éthoxy]-19-nor-pregna-1,3,5(10)-trièn-20-yn-17-ol,
- (17α)-3-[3-[méthyl(phénylméthyl) amino] propoxy]-19-nor-pregna-1,3,5(10)-trièn-20-yn-17-ol,
- (8α,9β,13α,14β)-3-[2-(diméthylamino) éthoxy]-estra-1,3,5(10)-trièn-17-one,
- (8α,9β,13α,14β)-3-[2-(diéthylamino) éthoxy]-estra-1,3,5(10)-trièn-17-one,
- (8α,9β,13α,14β)-3-[2-(diméthylamino) éthoxy]-estra-1,3,5(10)-triène,
- (8α,9β,13α,14β)-3-[2-(diéthylamino) éthoxy]-estra-1,3,5(10)-triène,
- chlorhydrate de (8α,9β,13α,14β)-3-[2(diéthylamino) éthoxy]-estra-1,3,5(10)-triène,
- (8α,9β,13α,14β,17α)-3-[2-(diéthylamino) éthoxy]-estra-1,3,5(10)-trièn-17-ol,
- (13α) -3-[2-(diméthylamino) éthoxy]-estra-1,3,5(10)-trièn-17-one,
- (13α)-3-[2-(diéthylamino) éthoxy]-estra-1,3,5(10)-trièn-17-one,
- (13α)-3-[2-(diméthylamino) éthoxy]-estra-1,3,5(10)-triène,

- (13α)-3-[2-(diéthylamino) éthoxy]-estra-1,3,5(10)-triène.

12. Application des composés de formule (I) tels que définis aux revendications 1 à 11 à titre de contraceptif masculin, étant entendu que l'utilisation de la contraception à titre privé est exclue.

**Patentansprüche**

1. Verwendung von Verbindungen der Formel (I):

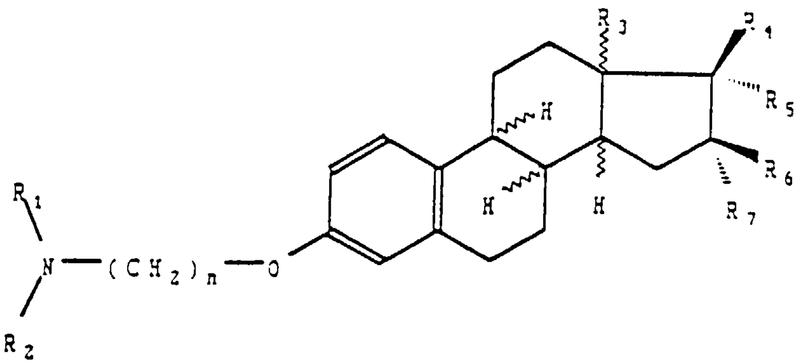

(I)

worin $R_1$ und $R_2$, die gleich oder verschieden sein können, einen Alkylrest mit 1 bis 8 Kohlenstoffatomen oder einen Benzylrest bedeuten oder zusammen mit dem Stickstoff, an den sie gebunden sind, einen 5- oder 6-gliedrigen gesättigten Heterozyklus mit gegebenenfalls einem weiteren Heteroatom, ausgewählt aus Sauerstoff und Stickstoff, bilden, $R_3$ in Position α oder β eine Methylgruppe bezeichnet, n eine ganze Zahl zwischen 1 und 10 bedeutet und entweder $R_4$ und $R_5$ zusammen eine Oxogruppierung bilden oder $R_4$ ein Wasserstoffatom, eine Hydroxylgruppe oder Acyloxygruppe mit höchstens 12 Kohlenstoffatomen bedeutet und $R_5$ ein Wasserstoffatom, eine Hydroxylgruppe, einen Acyloxyrest mit höchstens 12 Kohlenstoffatomen, einen Alkylrest, einen Alkenylrest oder einen Alkinylrest mit jeweils höchstens 8 Kohlenstoffatomen bedeutet, $R_6$ und $R_7$ zusammen eine Oxogruppierung bilden oder gleich oder verschieden sind und ein Wasserstoffatom, eine Hydroxylgruppe oder einen Acyloxyrest mit höchstens 12 Kohlenstoffatomen bedeuten, oder $R_5$ und $R_6$ zusammen eine Doppelbindung bilden und $R_4$ und $R_7$ Wasserstoffatome sind, sowie ihre Additionssalze mit pharmazeutischen verträglichen Säuren zur Herstellung eines Medikaments zur Kontrolle der Fertilität und insbesondere zur Kontrolle der männlichen Fertilität, ausgenommen Kontrazeption.

2. Verwendung nach Anspruch 1 der Verbindungen der allgemeinen Formel (I), wie in Anspruch 1 definiert, worin $R_3$ eine Methylgruppe in Position β ist, sowie der Additionssalze davon mit pharmazeutisch verträglichen Säuren.

3. Verwendung nach Anspruch 1 der Verbindungen der allgemeinen Formel (I), wie in Anspruch 1 oder 2 definiert, der allgemeinen Formel ($I_A$)

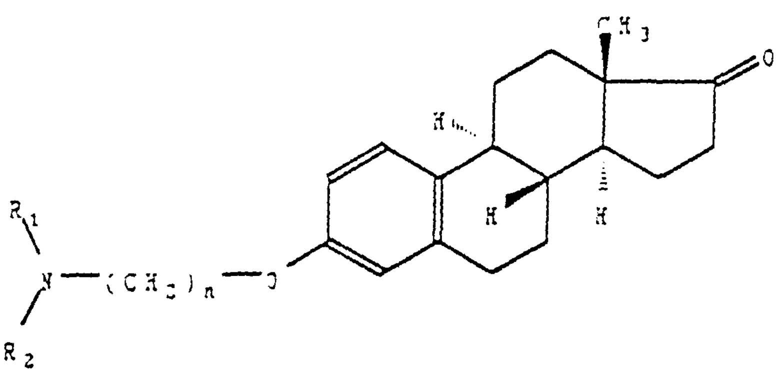

($I_A$)

worin $R_1$, $R_2$ und n wie in Anspruch 1 definiert sind, sowie der Additionssalze davon mit pharmazeutisch verträglichen Säuren.

**4.** Verwendung nach Anspruch 1 der Verbindungen der allgemeinen Formel (I), wie in Anspruch 1 oder 2 definiert, der allgemeinen Formel ($I_B$)

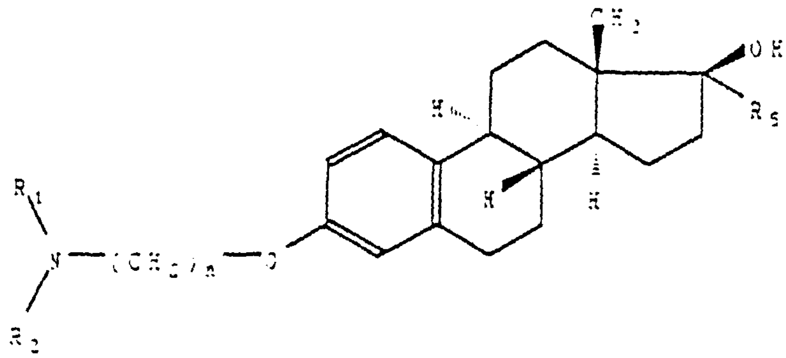

($I_B$)

worin $R_1$, $R_2$, $R_5$ und n wie in Anspruch 1 definiert sind, sowie der Additionssalze davon mit pharmazeutisch verträglichen Säuren.

**5.** Verwendung nach Anspruch 1 der Verbindungen der allgemeinen Formel (I), wie in Anspruch 1 definiert, der allgemeinen Formel ($I_C$)

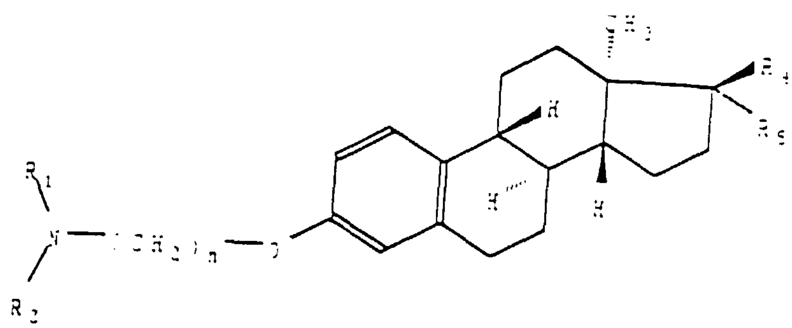

($I_C$)

worin $R_1$ und $R_2$, die gleich oder verschieden sind, einen Alkylrest mit 1 bis 8 Kohlenstoffatomen bedeuten, n wie vorstehend definiert ist und

a) entweder $R_4$ ein Wasserstoffatom bedeutet und $R_5$ eine Hydroxylgruppe bedeutet,
b) oder $R_4$ und $R_5$ zusammen eine Oxogruppe bilden,
c) oder $R_4$ und $R_5$ ein Wasserstoffatom bedeuten,

sowie ihrer Additionssalze mit pharmazeutisch verträglichen Säuren.

**6.** Verwendung nach Anspruch 1 der Verbindungen der allgemeinen Formel (I), wie in Anspruch 1 oder 2 definiert, der allgemeinen Formel ($I_D$)

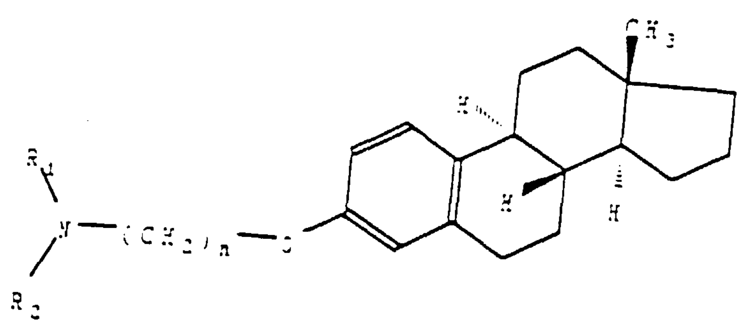

($I_D$)

worin $R_1$, $R_2$ und n wie in Anspruch 1 definiert sind, sowie ihrer Additionssalze mit pharmazeutisch verträglichen Säuren.

**7.** Verwendung nach Anspruch 1 der Verbindungen der allgemeinen Formel (I), wie in Anspruch 1 oder 2 definiert, der allgemeinen Formel ($I_E$)

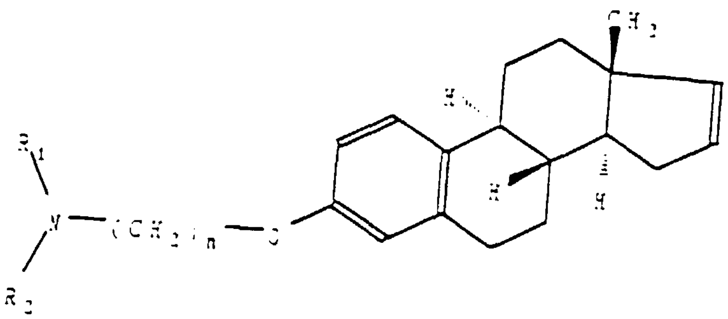

($I_E$)

worin $R_1$, $R_2$ und n wie in Anspruch 1 definiert sind, sowie ihrer Additionssalze mit pharmazeutisch verträglichen Säuren.

**8.** Verwendung nach Anspruch 1 der Verbindungen der allgemeinen Formel (I), wie in Anspruch 1 definiert, der allgemeinen Formel ($I_F$)

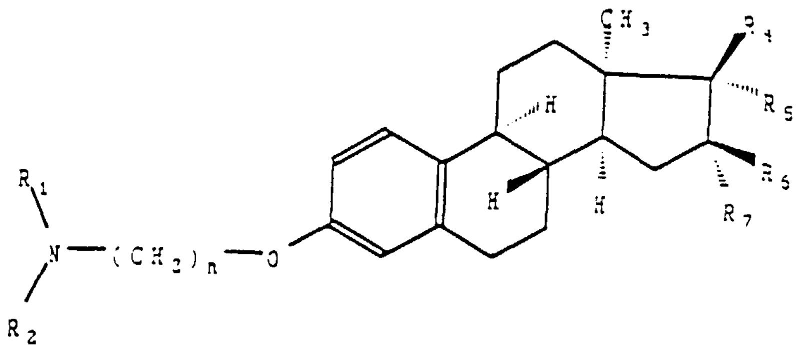

($I_F$)

worin $R_1$, $R_2$ und n wie in Anspruch 1 definiert sind und <u>entweder</u> $R_4$ und $R_5$ zusammen eine Oxogruppe bilden oder gleich oder verschieden sind und ein Wasserstoffatom, eine Hydroxylgruppe oder einen Acyloxyrest mit höchstens 12 Kohlenstoffatomen bedeuten, $R_6$ und $R_7$ zusammen eine Oxogruppe bilden oder gleich oder verschieden sind und ein Wasserstoffatom, eine Hydroxylgruppe oder eine Acyloxygruppe mit höchstens 12 Kohlenstoffatomen bedeuten, <u>oder</u> $R_5$ und $R_6$ zusammen eine Doppelbindung bilden und $R_4$ und $R_7$ Wasserstoffatome sind, sowie ihrer Additionssalze mit pharmazeutisch verträglichen Säuren.

**9.** Verwendung nach Anspruch 1 der Verbindungen der allgemeinen Formel (I), wie in einem der Ansprüche 1 bis 8 definiert, worin $R_1$ oder $R_2$, die gleich sind, eine Methyl- oder Ethylgruppe bedeuten, sowie ihrer Additionssalze mit pharmazeutisch verträglichen Säuren.

**10.** Verwendung nach Anspruch 1 der Verbindung der allgemeinen Formel (I), wie in einem der Ansprüche 1 bis 9 definiert, worin n den Wert 2 hat, sowie ihrer Additions-salze mit pharmazeutisch verträglichen Säuren.

**11.** Verwendung nach Anspruch 1 der Verbindung der allgmeinen Formel (I), wie in Anspruch 1 definiert, nämlich:

- 3-[2-Dimethylamino)ethoxy]estra-1,3,5(10)-trien-17-on,
- 3-[2-Diethylamino)ethoxy]estra-1,3,5(10)-trien-17-on,
- 3-[2-(Dimethylamino)ethoxy]estra-1,3,5(10)-trien-17-on-chlorhydrat,
- 3-[2-(Diethylamino)ethoxy]estra-1,3,5(10)-trien-17-on-chlorhydrat,
- 3-[2-(1-Piperidinyl)ethoxy]estra-1,3,5(10)-trien-17-on,

- 3-[2-(4-Morpholinyl)ethoxy]estra-1,3,5(10)-trien-17-on,
- 3-[3-(Dimethylamino)propoxy]estra-1,3,5(10)-trien-17-on,
- 3-[3-(Diethylamino)propoxy]estra-1,3,5(10)-trien-17-on,
- 3-[4-(Dimethylamino)butoxy]estra-1,3,5(10)-trien-17-on,
- 3-[5-(Dimethylamino)pentyloxy]estra-1,3,5(10)-trien-17-on,
- 3-[5-(Diethylamino)pentyloxy]estra-1,3,5(10)-trien-17-on,
- 3-[10-(Dimethylamino)decyloxy]estra-1,3,5(10)-trien-17-on,
- 3-[2-[2-Methyl(phenylmethyl)amino]ethoxy]estra-1,3,5(10)-trien-17-on,
- 3-[3-[Bis(phenylmethyl)amino]propoxy]estra-1,3,5(10)-trien-17-on,
- (17β)-3-[2-[Methyl(phenylmethyl)amino]ethoxy]estra-1,3,5(10)-trien-17-ol,
- (17β)-3-[3-[Dimethylamino)propoxy]estra-1,3,5(10)-trien-17-ol,
- (17β)-3-[3-(Diethylamino)propoxy]estra-1,3,5(10)-trien-17-ol,
- (17α)-3-[3-(Diethylamino)propoxy]estra-1,3,5(10)-trien-17-ol,
- (17α)-3-[2-(Dimethylamino)ethoxy]estra-1,3,5(10)-trien-17-ol,
- (17α)-3-[2-(Diethylamino)ethoxy]estra-1,3,5(10)-trien-17-ol,
- 3-[2-(Dimethylamino)ethoxy]estra-1,3,5(10)-trien-16-on,
- 3-[2-(Diethylamino)ethoxy]estra-1,3,5(10)-trien-16-on,
- 3-[2-(Diethylamino)ethoxy]estra-1,3,5(10)-trien-16-on-chlorhydrat,
- 3-[2-(Dimethylamino)ethoxy]estra-1,3,5(10)-trien,
- 3-[2-(Diethylamino)ethoxy]estra-1,3,5(10)-trien,
- 3-[2-(1-Piperidinyl)ethoxy]estra-1,3,5(10)-trien,
- 3-[3-(1-Piperidinyl)propoxy]estra-1,3,5(10)-trien,
- 3-[2-(Diethylamino)ethoxy]estra-1,3,5(10)-trienchlorhydrat,
- 3-[2-(4-Morpholinyl)ethoxy]estra-1,3,5(10)-trien,
- 3-[2-(Dimethylamino)ethoxy]estra-1,3,5(10),16-tetraen,
- 3-[2-(Diethylamino)ethoxy]estra-1,3,5(10),16-tetraen,
- (17α)-3-[3-(Diethylamino)propoxy]-17-methylestra-1,3,5(10)-trien-17-ol,
- (17α)-3-[2-(Dimethylamino)ethoxy]-17-methylestra-1,3,5(10)-trien-17-ol,
- (17α)-3-[3-(Dimethylamino)propoxy]-19-norpregna-1,3,5(10)-trien-17-ol,
- (17α)-3-[2-(Dimethylamino)ethoxy]-19-norpregna-1,3,5(10)-trien-17-ol,
- (17α)-3-[2-[Methyl(phenylmethyl)amino]ethoxy]-19-norpreg-na-1,3,5(10)-trien-17-ol,
- (17α)-3-[3-(Dimethylamino)propoxy]-17-propylestra-1,3,5(10)-trien-17-ol,
- (17α)-3-[3-(Dimethylamino)propoxy]-17-propylestra-1,3,5(10)-trien-17-olchlorhydrat,
- (17α)-3-[3-(Dimethylamino)propoxy]-17-(1-propenyl)estra-1,3,5(10)-trien-17-ol,
- (17α)-3-[3-(Dimethylamino)propoxy]-17-(1-propenyl)estra-1,3,5(10)-trien-17-olchlorhydrat,
- (17α)-3-[2-(Dimethylamino)ethoxy]-19-norpregna-1,3,5(10),20-tetraen-17-ol,
- (17α)-3-[3-(Dimethylamino)propoxy]-19-norpregna-1,3,5(10),20-tetraen-17-ol,
- (17α)-3-[2-(Dimethylamino)ethoxy]-19-norpregna1,3,5(10)-trien-20-in-17-ol,
- (17α)-3-[3-(Dimethylamino)propoxy]-19-norpregna1,3,5(10)-trien-20-in-17-ol,
- (17α)-3-[3-(Dimethylamino)propoxy]-19-norpregna1,3,5(10)-trien-20-in-17-olchlorhydrat,
- (17α)-3-[2-(Diethylamino)ethoxy]-19-norpregna-1,3,5(10)-trien-20-in-17-ol,
- (17α)-3-[3-(Diethylamino)propoxy]-19-norpregna-1,3,5(10)-trien-20-in-17-ol,
- (17α)-3-[2-[Methyl(phenylmethyl)amino]ethoxy]-19-nor-pregna-1,3,5(10)-trien-20-yn-17-ol,
- (17α)-3-[2-[Methyl(phenylmethyl)amino]ethoxy]-19-norpregna-1,3,5(10)-trien-20-yn-17-olchlorhydrat,
- (17α)-3-[3-[Methyl(phenylmethyl)amino]propoxy]-19-norpregna-1,3,5(10)-trien-20-yn-17-ol,
- (8α,9β,13α,14β)-3-[2-(Dimethylamino)ethoxy]estra1,3,5(10)-trien-17-on,
- (8α,9β,13α,14β)-3-[2-(Diethylamino)ethoxy]estra-1,3,5(10)-trien-17-on,
- (8α,9β,13α,14β)-3-[2-(Dimethylamino)ethoxy]estra-1,3,5(10)-trien,
- (8α,9β,13α,14β)-3-[2-(Diethylamino)ethoxy]estra-1,3,5(10)-trien,
- (8α,9β,13α,14β)-3-[2-(Diethylamino)ethoxy]estra-1,3,5(10)-trienchlorhydrat,
- (8α,9β,13α,14β,17α)-3-[2-(Diethylamino)ethoxy]estra-1,3,5(10)-trien-17-ol,
- (13α)-3-[2-(Dimethylamino)ethoxy]estra-1,3,5(10)-trien-17-on,
- (13α)-3-[2-(Diethylamino)ethoxy]estra-1,3,5(10)-trien-17-on,
- (13α)-3-[2-(Dimethylamino)ethoxy]estra-1,3,5(10)-trien,
- (13α)-3-[2-(Diethylamino)ethoxy]estra-1,3,5(10)-trien.

**12.** Verwendung der Verbindungen der Formel (I), wie in den Ansprüchen 1 bis 11 definiert, als männliches Kontrazeptivum mit der Maßgabe, dass die kontrazeptive Verwendung zu privaten Zwecken ausgeschlossen ist.

## Claims

1. The use of compounds of the formula (I):

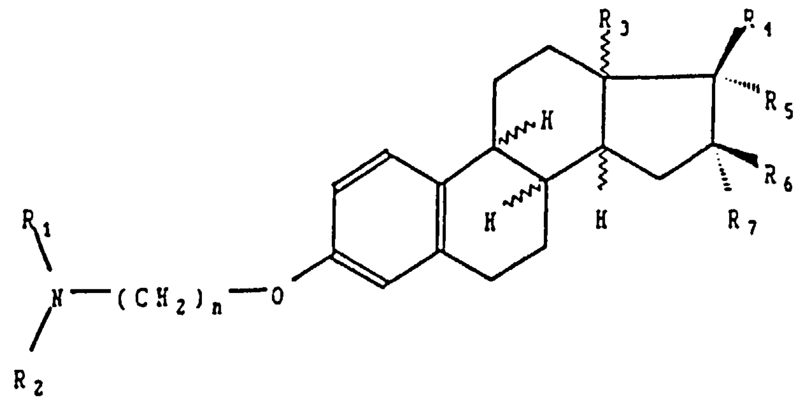

(I)

in which $R_1$ and $R_2$ are identical or different and represent an alkyl grouping having 1 to 8 carbon atoms or a benzyl grouping, or together with the nitrogen atom to which they are bonded form a saturated 5- or 6-membered heterocyclic radical optionally containing another heteroatom chosen from oxygen and nitrogen, $R_3$ in the α- or β-position represents a methyl grouping, n designates an integer between 2 and 10 and either $R_4$ and $R_5$ together form an oxo grouping, or $R_4$ represents a hydrogen atom, a hydroxyl grouping or acyloxy having at most 12 carbon atoms and $R_5$ represents a hydrogen atom, a hydroxyl grouping, acyloxy having at most 12 carbon atoms, alkyl, alkenyl or alkinyl each having at most 8 carbon atoms, $R_6$ and $R_7$ together form an oxo grouping or are identical or different and represent a hydrogen atom, a hydroxyl grouping or acyloxy having at most 12 carbon atoms, or $R_5$ and $R_6$ together form a double bond and $R_4$ and $R_7$ are hydrogen atoms, and their addition salts with pharmaceutically acceptable acids to obtain a medicament intended for control of fertility, and in particular for control of male fertility, with the exception of contraception.

2. Use according to claim 1 of compounds of the general formula (I) as defined in claim 1, in which:
$R_3$ is a methyl grouping in the p-position, and their addition salts with pharmaceutically acceptable acids.

3. The use according to claim 1 of compounds of the general (I) as defined in claim 1 or 2 corresponding to the general formula ($I_A$) :

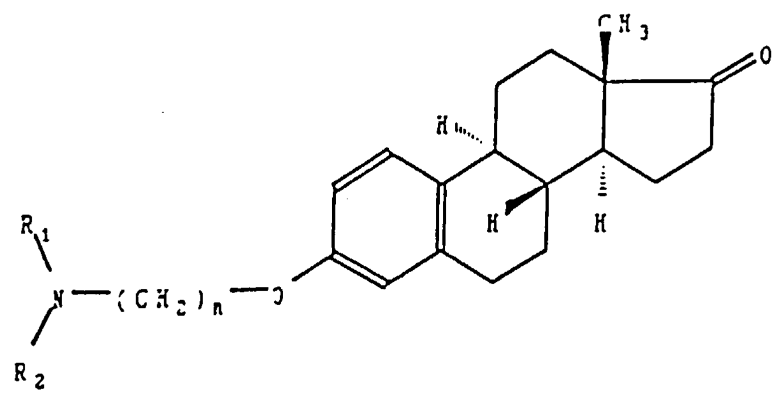

($I_A$)

in which $R_1$, $R_2$ and n are as defined in claim 1, and their addition salts with pharmaceutically acceptable acids.

4. The use according to claim 1 of compounds of the general (I) as defined in claim 1 or 2 corresponding to the general formula ($I_B$) :

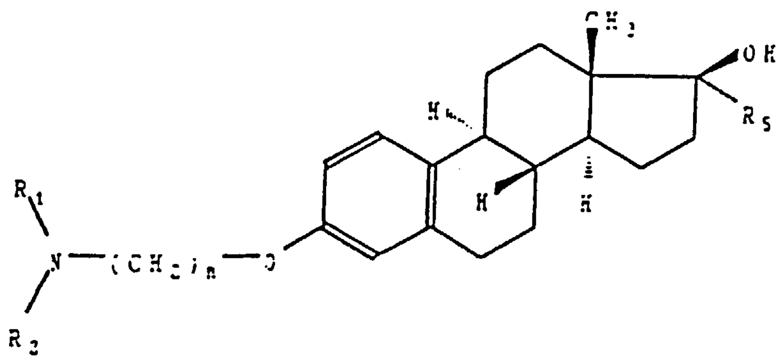

($I_B$)

in which $R_1$, $R_2$, $R_5$ and n are as defined in claim 1, and their addition salts with pharmaceutically acceptable acids.

**5.** The use according to claim 1 of compounds of the general formula (I) as defined in claim 1 corresponding to the general formula ($I_C$) :

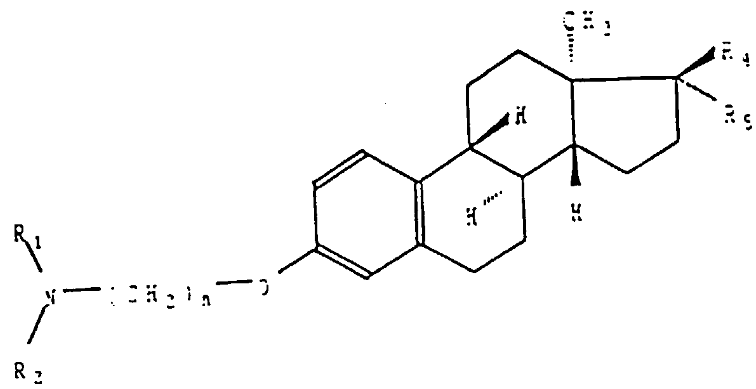

($I_C$)

in which $R_1$ and $R_2$ are identical or different and represent an alkyl grouping having 1 to 8 carbon atoms, n is as defined above and

a) either $R_4$ represents a hydrogen atom and $R_5$ represents a hydroxyl grouping,
b) or $R_4$ and $R_5$ together form an oxo grouping,
c) or $R_4$ and $R_5$ represent a hydrogen atom,

and their addition salts with pharmaceutically acceptable acids.

**6.** The use according to claim 1 of compounds of the general (I) as defined in claim 1 or 2 corresponding to the general formula ($I_D$) :

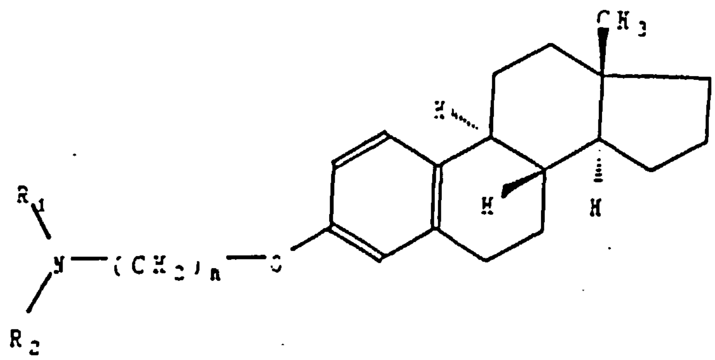

(I_D)

in which $R_1$, $R_2$ and n are as defined in claim 1, and their addition salts with pharmaceutically acceptable acids.

**7.** The use according to claim 1 of compounds of the general (I) as defined in claim 1 or 2 corresponding to the general formula ($I_E$) :

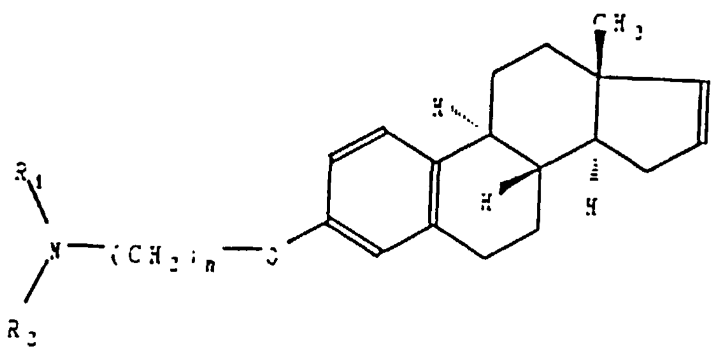

($I_E$)

in which $R_1$, $R_2$ and n are as defined in claim 1, and their addition salts with pharmaceutically acceptable acids.

**8.** The use according to claim 1 of compounds of the general formula (I) as defined in claim 1 corresponding to the general formula ($I_F$) :

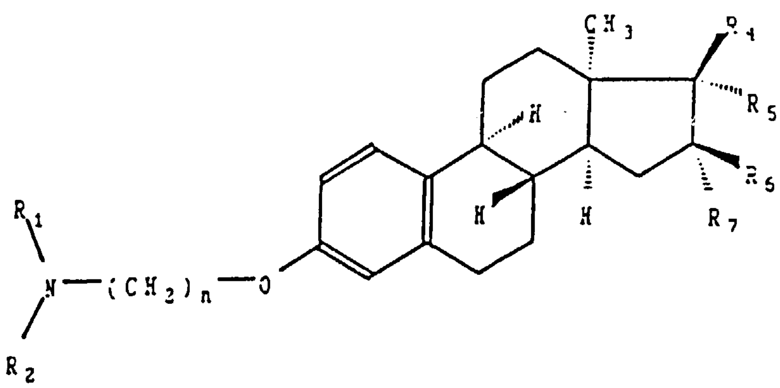

($I_F$)

in which $R_1$, $R_2$ and n are as defined above and <u>either</u> $R_4$ and $R_5$ together form an oxo grouping or are identical or different and represent a hydrogen atom, a hydroxyl grouping or acyloxy having at most 12 carbon atoms, $R_6$ and $R_7$ together form an oxo grouping or are identical or different and represent a hydrogen atom, a hydroxyl grouping or acyloxy having at most 12 carbon atoms, <u>or</u> $R_5$ and $R_6$ together form a double bond and $R_4$ and $R_7$ are hydrogen atoms, and their addition salts with pharmaceutically acceptable acids.

**9.** The use according to claim 1 of compounds of the general formula (I) as defined in any one of claims 1 to 8, in which $R_1$ and $R_2$ are identical and represent methyl or ethyl, and their addition salts with pharmaceutically acceptable acids.

**10.** The use according to claim 1 of compounds of the general formula (I) as defined in any one of claims 1 to 9, in which n is equal to 2, and their addition salts with pharmaceutically acceptable acids.

**11.** The use according to claim 1 of the following compounds of the general formula (I) as defined in claim 1:

- 3-[2-(dimethylamino)ethoxy]-oestra-1,3,5(10)-trien-17-one,
- 3-[2-(diethylamino)ethoxy]-oestra-1,3,5(10)-trien-17-one,
- hydrochloride of 3-[2-dimethylamino)ethoxy]-oestra-1,3,5(10)-trien-17-one,
- hydrochloride of 3-[2-(diethylamino)ethoxy]-oestra-1,3,5(10)-trien-17-one,
- 3-[2-(1-piperidinyl)ethoxy]-oestra-1,3,5(10)-trien-17-one,
- 3-[2-(4-morpholinyl)ethoxy]-oestra-1,3,5(10)-trien-17-one,
- 3-[3-(dimethylamino)propoxy]-oestra-1,3,5(10)-trien-17-one,
- 3-[3-(diethylamino)propoxy]-oestra-1,3,5(10)-trien-17-one,
- 3-(4-(dimethylamino)butoxy]-oestra-1,3,5(10)-trien-17-one,
- 3-[5-(dimethylamino)pentyloxy]-oestra-1,3,5(10)-trien-17-one,
- 3-[5-(diethylamino)pentyloxy]-oestra-1,3,5(10)-trien-17-one,
- 3-[10-(dimethylamino)decyloxy]-oestra-1,3,5(10)-trien-17-one,
- 3-[2-(methyl(phenylmethyl)amino]ethoxy]-oestra-1,3,5(10)-trien-17-one,
- 3-[3-[bis(phenylmethyl)amino]propoxy]-oestra-1,3,5(10)-trien-17-one,
- (17β)-3-[2-[methyl(phenylmethyl)amino]ethoxy]-oestra-1,3,5(10)-trien-17-ol,
- (17β)-3-[3-(dimethylamino)propoxy]-oestra-1,3,5(10)trien-17-ol,
- (17β)-3-[3-(diethylamino)propoxy]-oestra-1,3,5(10)trien-17-ol,
- (17α)-3-[3-(diethylamino)propoxy]-oestra-1,3,5(10)-trien-17-ol,
- (17α)-3-[2-(dimethylamino)ethoxy]-oestra-1,3,5(10)-trien-17-ol,
- (17α)-3-[2-(diethylamino)ethoxy]-oestra-1,3,5(10)-trien-17-ol,
- 3-[2-(dimethylamino)ethoxy]-oestra-1,3,5(10)-trien-16-one,
- 3-[2-(diethylamino)ethoxy]-oestra-1,3,5(10)-trien-16-one,
- hydrochloride of 3-[2-(diethylamino)ethoxy]-oestra-1,3,5(10)-trien-16-one,
- 3-[2-(dimethylamino)ethoxy]-oestra-1,3,5(10)-triene,
- 3-[2-(diethylamino)ethoxy]-oestra-1,3,5(10)-triene,
- 3-[2-(1-piperidinyl)ethoxy]-oestra-1,3,5(10)-triene,
- 3-[3-(1-piperidinyl)propoxy]-oestra-1,3,5(10)-triene,
- hydrochloride of 3-[2-(diethylamino)ethoxy]-oestra-1,3,5(10)-triene,
- 3-[2-(4-morpholinyl)ethoxy]-oestra-1,3,5(10)-triene,
- 3-[2-(dimethylamino)ethoxy)]-oestra-1,3,5(10),16-tetraene,
- 3-[2-(diethylamino)ethoxy)]-oestra-1,3,5(10),16-tetraene,
- (17α)-3-[3-dimethylamino)propoxy]-17-methyl-oestra-1,3,5(10)-trien-17-ol,
- (17α)-3-[2-(dimethylamino)ethoxy]-17-methyl-oestra-1,3,5(10)-trien-17-ol,
- (17α)-3-[3-(dimethylamino)propoxy]-19-nor-pregna-1,3,5(10)-trien-17-ol,
- (17α)-3-[2-(dimethylamino)ethoxy]-19-nor-pregna-1,3,5(10)-trien-17-ol,
- (17α)-3-[2-[methyl(phenylmethyl)amino]ethoxy]-19-nor-pregna-1,3,5(10)-trien-17-ol,
- (17α)-3-[3-(dimethylamino)propoxy]-17-propyl-oestra-1,3,5(10)-trien-17-ol,
- hydrochloride of (17α)-3-[3-(dimethylamino)propoxy]-17-propyl-oestra-1,3,5(10)-trien-17-ol,
- (17α)-3-[3-(dimethylamino)propoxy]-17-(1-propenyl)-oestra-1,3,5(10)-trien-17-ol,
- hydrochloride of (17α)-3-[3-(dimethylamino)propoxy]-17-(1-propenyl)-oestra-1,3,5(10)-trien-17-ol,
- (17α)-3-[2-(dimethylamino)ethoxy)]-19-nor-pregna-1,3,5(10),20-tetraen-17-ol,
- (17α)-3-[3-(dimethylamino)propoxy]-19-nor-pregna-1,3,5(10),20-tetraen-17-ol,
- (17α)-3-[2-(dimethylamino)ethoxy]-19-nor-pregna-1,3,5(10)-trien-20-in-17-ol,
- (17α)-3-[3-(dimethylamino)propoxy]-19-nor-pregna-1,3,5(10)-trien-20-in-17-ol,
- hydrochloride of (17α)-3-[3-(dimethylamino)propoxy]-19-nor-pregna-1,3,5(10)-trien-20-in-17-ol,
- (17α)-3-[2-(diethylamino)ethoxy]-19-nor-pregna-1,3,5(10)-trien-20-in-17-ol,
- (17α)-3-[3-(diethylamino)propoxy]-19-nor-pregna-1,3,5(10)-trien-20-in-17-ol,
- (17α)-3-[20-[methyl(phenylmethyl)amino]ethoxy]-19-nor-pregna-1,3,5(10)-trien-2-in-17-ol,
- hydrochloride of (17α)-3-[2-[methyl(phenylmethyl)amino]ethoxy]-19-nor-pregna-1,3,5(10)-trien-20-in-17-ol,

- (17α)-3-[3-[methyl(phenylmethyl)amino]propoxy]-19-nor-pregna-1,3,5(10)-trien-20-in-17-ol,
- (8α,9β,13α,14β)-3-[2-(dimethylamino)ethoxy]-oestra-1,3,5(10)-trien-17-one,
- (8α,9β,13α,14β)-3-[2-(diethylamino)ethoxy]-oestra-1,3,5(10)-trien-17-one,
- (8α,9β,13α,14β)-3-[2-(dimethylamino)ethoxy]-oestra-1,3,5(10)-triene,
- (8α,9β,13α,14β)-3-[2-(diethylamino)ethoxy]-oestra-1,3,5(10)-triene,
- hydrochloride of (8α,9β,13α,14β)-3-[2(diethylamino)ethoxy]-oestra-1,3,5(10)-triene,
- (8α,9β,13α,14β,17α)-3-[2-(diethylamino)ethoxy]-oestra-1,3,5(10)-trien-17-ol,
- (13α)-3-[2-(dimethylamino)ethoxy]-oestra-1,3,5(10)-trien-17-one,
- (13α)-3-[2-(diethylamino)ethoxy]-oestra-1,3,5(10)-trien-17-one,
- (13α)-3-[2-(dimethylamino)ethoxy]-oestra-1,3,5(100-triene,
- (13α)-3-[2-(diethylamino)ethoxy]-oestra-1,3,5(10)-triene.

**12.** Use of compounds of the formula (I) as defined in claims 1 to 11 as a male contraceptive, it being understood that the use of personal contraception is excluded.